# EUROPEAN PATENT APPLICATION

(11) **EP 4 684 811 A1**
(43) Date of publication of application: **28.01.2026**
(21) Application number: 24190543.9
(22) Date of filing: 24.07.2024
(51) Int. Cl.: A61L 27/02, A61L 27/12, A61L 27/54

(54) **NOVEL TREATMENT REGIMEN AND METHODS IN BONE HEALING**

(71) Applicant: Moroxite AB, 298 31 Tollarp (SE)
(72) Inventor: LIDGREN, Lars, 298 31 Tollarp (SE); RAINA, Deepak, 298 31 Tollarp (SE); TÄGIL, Magnus, 298 31 Tollarp (SE)
(74) Representative: Zacco Sweden AB

(57) **Abstract**

Present invention relates to a novel ceramic carrier and novel approaches in bone healing.

## Description

### TECHNICAL FIELD

Present invention relates to novel approaches in bone healing. Specifically, present invention relates to novel compositions which may comprise a ceramic component. According to the invention, the composition may also comprise one or more bone morphogenetic proteins (BMPs) and may further be supplied with a one or more antibiotic compounds or compounds capable of acting as an antibiotic compound. Present invention also relates to a novel process of preparing the compositions according to the invention.

### BACKGROUND ART

Spinal fusion is a standard treatment for various spinal disorders, including trauma, deformity, degenerative disorders, tumours and infections, with more than 400,000 surgical procedures performed annually in the USA alone. The total number of spinal fusion procedures has been on the rise over the past few decades globally, mainly due to improvements in surgical techniques, better implants and expansion of surgical indications. Posterolateral spinal fusion (PLF) is a common surgical procedure to treat spinal instability. The permanent stability of the procedure relies on bony fusion achieved by bone grafting between the transverse processes. A recent review reported that PLF failure rate ranges between 10 % and 55 %, impacting the final functional outcome. The availability of autologous bone is limited and the PLF procedure requires large volumes of bone graft. Furthermore, harvesting autologous bone is associated with donor site-related complications, including pain, infection and sensory abnormalities. Allografts are attractive alternatives to autografts, but they carry potential risks of disease transmission and immunogenicity. Therefore, the development of an off-the-shelf, synthetic bone graft substitute with high bone formation capacity is a promising treatment modality for fusion.

In 2002, rhBMP-2, a lyophilized powder co-packed with a solvent, and added to a collagen-based sponge was approved by the Food and Drug Administration (FDA) for clinical use in single-stage PLF and fusion (ALIF) within a specific interbody cage. The usage has increased over the past few decades. Recent reviews found no clear advantage of using rhBMP-2 compared to iliac crest bone graft and side effects associated with rhBMP-2 were reported, including inflammation, radiculopathy, ectopic bone formation, osteolysis, genitourinary events, and wound complications, which all limited its clinical application in spinal surgery. Numerous reports indicate that not only supra-physiological addition of rhBMP-2 but also carrier-related with absorbable collagen sponges (ACS) leading to burst release as the main cause of the adverse events.

Moreover, the pharmacokinetics of BMP release from the carrier are critical in terms of safety.

At present, BMP carriers include natural polymers, synthetic polymers, inorganic materials, and composites that are in preclinical research and have not been translated into clinically useful applications. The surrounding chemical environment and mechanical stimuli determine the actual bone growth and morphology. It is important to realise that a second rebound osteoclast effect especially with unfavourable non weight bearing conditions may occur and that this second resorption could be prevented in selected cases by giving systemic bisphosphonates. A low dose of Zoledronic acid (1 mg) peri or postoperative can significantly reduce associated systemic and kidney complications but still be effective in preventing secondary osteoclast resorption.

The challenge to develop an optimal controlled-release carrier acting as an efficient scaffold for delivery of rhBMP-2 still remains and there is a need to overcome the difficulties and observed adverse effects.

Also, a documented complication in instrumented spinal fusion is infection occurring in 10-30% of the patients. As the typical patient tends to be an older individual with substantial comorbidity, an infection often results in prolonged and repeated surgeries, secondary complications, chronic morbidity, and even mortality. In addition, emerging bacterial resistance poses a major threat.

A further complication arises as both BMP-2 and certain antibiotics are destroyed or affected by a sterilisation procedure and they need to be sterile produced, filled and packed.

Present invention addresses the above discussed problems and provides a solution thereto.

### SUMMARY OF THE INVENTION

Present invention relates to a vehicle or a carrier, which may be employed as an implant in the context of bone healing.

Moreover, present invention relates to said vehicle or carrier comprising one or more BMPs and optionally also one or more antibiotic compounds or compounds capable of acting as an antibiotic compound.

Present invention also relates to a composition comprising a vehicle or a carrier, one or more BMPs and optionally also one or more antibiotic compounds or compounds capable of acting as an antibiotic compound.

Present invention also relates to a process of preparing the aforementioned composition.

Present invention thus provides one or more of the following advantages;
a) avoiding undesirable side effects observed when employing BMPs in medical treatment,
b) employing lower doses of the employed BMPs and still obtain desirable and even superior results in terms of bone healing and bone formation.
c) providing methods of preparation enabling industrial production of the composition according to the invention.

In one aspect, the carrier or vehicle may comprise hydroxyapatite (HA) and/or calcium phosphate (CaP).

In a further aspect, the carrier may comprise at least one additional component which may be e.g. calcium sulphate (CaS). However, the carrier may comprise any other ceramic or non-ceramic components.

In yet a further aspect, the carrier may comprise calcium sulphate (CaS). Consequently, the carrier according to the invention may comprise at least one of HA, CaP, or CaS or any combinations thereof,
In one aspect, the carrier according to the invention may comprise other components, such as e.g. autologous bone.

In a further aspect, the carrier may comprise one or more BMPs such as e.g. recombinant human BMPs (rhBMPs) such as e.g. rhBMP-2 and/or rhBMP-7.

The composition according to the invention may find use in in orthopaedic applications such as spinal fusions, nonunions, and oral surgery or in any context relating to bone healing or bone formation in a subject.

As also indicated herein, present invention also relates to a novel method of preparing a composition according to the invention. The method may entail soaking or impregnating the carrier or vehicle with e.g. one or more BMPs and/or antibiotic compounds.

Furthermore, present invention relates to use of the composition according to the invention for bone healing or regeneration in a subject. The subject may be a human or an animal.

In another aspect, present invention relates to the composition according to the invention for use in bone healing or bone regeneration in a subject. The subject may be a human or an animal.

As is evident to a person skilled in the art, the composition according to the invention may be used in the context of any type of bone healing or bone regeneration, such as e.g. spinal fusion or may be used in the context of healing a bone fracture of any kind, such as e.g. a hip fracture, fracture in the femur etc.

Further aspects of the invention will be discussed below.

### BRIEF DESCRIPTION OF THE DRAWINGS

**Figure 1** illustrates the final setting time of a biphasic ceramic material is prolonged by the addition of BMP-2.
**Figure 2** illustrates the 1 mm x 1 mm beads casted in a cylindrical shape.
**Figure 3** illustrates the rat critical femoral defect filled with 1 mm x 1 mm pellets according to the invention.
**Figure 4** illustrates the micro-CT images taken 6-weeks after surgery indicating complete defect bridging in the BMP-2 group (right) and no healing in the empty control (left).
**Figure 5** illustrates the preparation of a mixture of autologous bone and beads according to the invention for in-vivo implantation.
**Figure 6** illustrates the implantation of beads according to the invention as a bridge between the decorticated transverse processes of L4 and L5 in a rabbit (left) visualized radiographically (right) immediately post-surgery.
**Figure 7** illustrates the radiographic evaluation of the spinal fusion after 6-weeks of surgery in a rabbit posterolateral spinal fusion model. Dashed box indicates the area of L4-L5 spine where the fusion procedure was carried out while the black arrows indicate radio dense areas between the transverse processes of L4 and L5.
**Figure 8** illustrates the in-vitro experiments showing inhibition of S. aureus growth around beads according to the invention after being soak loaded with TET (tetracycline) and RIF (rifampicin) for different time intervals.
**Figure 9** illustrates the differences in the volume of saline absorbed by pellets according to the invention with and without the presence of vacuum.
**Figure 10** illustrates the stain penetration experiment demonstrating the advantage of using under pressure in enhancing liquid penetration in a microporous ceramic material. White areas show material not penetrated by the fluid while dark areas show areas penetrated by the fluid.

### DETAILED DESCRIPTION

Present invention relates to a carrier or vehicle or a matrix or bone extender, terminology which may be used interchangeably throughout the text. The carrier may comprise hydroxyapatite (HA) and/or calcium phosphate (CaP).

In one aspect, the carrier or vehicle may be may be hydroxyapatite ("HA" in present text) or calcium phosphate ("CaP" in present text) in any configuration or crystal structure. Merely for sake of completion, hydroxyapatite which may also be referred to as hydroxylapatite, has the formula Ca₅(PO₄)₃(OH), but may also be written as Ca₁₀(PO₄)₆(OH)₂ to denote that the crystal unit cell comprises two entities. In another aspect, calcium phosphate may be a salt comprising Ca²⁺ combined with either of PO₄⁻³, HPO₂⁻⁴, or H₂PO⁻⁴. Other non-limiting examples are monocalcium phosphate (Ca(H₂PO₄)₂ and Ca(H₂PO₄)₂(H₂O), or dicalcium phosphate (CaHPO₄(H₂O)₂ or CaHPO₄(H₂O)), or tricalcium phosphate (Ca₃(PO₄)₂), or octacalcium phospahate (Ca₈H₂(PO₄)₆·5H₂O). In another aspect, CaP may be intended to mean amorphous calcium phosphate, which is a glassy precipitate of variable composition that may be present in biological systems.

The carrier or vehicle may further comprise a second material, which may, or may not, be a ceramic material. The second material may comprise one component or may be mixtures of several components. The relation between the first material and the second material may be such that the second material is absorbed by the body of the subject at a higher rate than the first material. However, the material may have similar properties in terms of absorbance of the body as e.g. HA and/or CaP. Non-limiting examples of a second material is one or more of fibrogen, dextran, hyaluronic acid, alfatachycalciferol, calcium sulphate (hemi-hydrate and/or di-hydrate), collagen, various forms of cellulose such as carboxymethyl cellulose (CMC) or sodium-CMC etc. or other polysaccharides such as e.g. agarose. Other non-limiting examples are blood or blood derived components such as a fibrin clot.

In one aspect, the second material may comprise e.g. calcium sulphate (hemi-hydrate and/or di-hydrate) in any crystal configuration. Calcium sulphate may be abbreviated or denoted as "CaS" in present text. As also indicated herein, the carrier may comprise or consist of CaS, or may comprise of CaS without HA and/or CaP.

The particle size of HA and/or CaP may in principle be in any range, such as e.g in range of micro-particles or nanoparticles. The size of these particles may be e.g. less than 200 µm, such as less than 100 µm, less than 50 µm, less than 35 µm, less than 20 µm or less than 10 µm. Moreover, the particles may be between about 0.1 and about 50 µm. In another aspect the microparticles may be in range of about 1 µm to about 500 µm, such as .e.g about 1 µm to about 100 µm, about 1 µm to about 50 µm, about 1 µm to about 25 µm, about 1 µm to about 15 µm, about 1 µm to about 10 µm, or about 1 µm, about 5 µm, about 10 µm, about 15 µm, about 20 µm, about 25 µm, about 30 µm, about 35 µm, about 40 µm, about 45 µm, about 50 µm, about 75 µm, about 100 µm, about 500 µm etc.

In one aspect, the particles of the first material is in range of e.g. about 0,5 µm to about 50 µm.

In one aspect, the particles of the first material is in range of e.g. about 1 µm to about 25 µm.

In one aspect, the particles of the first material is in range of e.g. about 1 µm to about 10 µm.

In yet a further aspect, the particle size may be in range of e.g. about 1 nm to about 200 nanometres (nm), such as e.g. less than about 150 nm, less than about 100 nm, less than about 50 nm, less than about 45 nm, less than about 40 nm, less than 35 nm, less than about 30 nm, less than about 25 nm, less than about 20 nm, less than about 15 nm, or less than about 10 nm. In one aspect the particle size is in range of e.g. about 1 nm to about 100 nm. In a further aspect, the particles of the first material may be about 1 nm, such as e.g. 5 nm, such as e.g. about 10 nm, such as e.g. about 15 nm, such as e.g. about 20 nm, such as e.g. about 25 nm, such as e.g. about 30 nm, such as e.g. about 35 nm, such as e.g. about 40 nm, such as e.g. about 45 nm, such as e.g. about 50 nm, such as e.g. about 100 nm, such as e.g. about 150 nm, or such as e.g. about 200 nm.

In one aspect, the particle size may be in range of about 40 nm to about 100 nm.

In a further aspect, the particle size may be in range of 10 nm to about 50 nm.

In yet a further aspect, the particle sizes may be a mixture of both micro-sized particles and nano-sized particles such as e.g. a mixture of particles in size of about 0.5 µm to about 50 µm and 40 nm to about 100 nm.

As indicated above, the carrier may be bi-phasic, i.e. comprise HA and/or CaP and an additional further material such as e.g. CaS. However, the carrier may be tri-phasic, tetraphasic etc.

In one aspect, the particles of HA and/or CaP may be embedded into the second material, which may be e.g. CaS.

The carrier or vehicle may in principle have any shape such as e.g. beads, rods, granules, or pellets etc. The size of these elements should be such that they are suitable for administration to, near or into the site of bone in a subject. Such administration may be in any suitable standard form such as e.g. injection or depositing near and/or into the site of a bone structure in a subject.

The composition according to present invention may also comprise one or more Bone morphogenetic proteins ("BMPs" in present text). The BMP may be one or more BMPs and may be e.g. one or more of BMP1, BMP2, BMP3, BMP4, BMP5, BMP6, BMP7, BMP8a, BMP8b, BMP10, BMP11, BMP15 or any combinations thereof. The BMP may also be a recombinant protein, such as e.g. a recombinant human BMP (rhBMP), such as e.g. rhBMP-2 and/or rhBMP-7.

In one aspect, the BMP may be rhBMP-2.

In another aspect, the BMP may be rhBMP-7.

In a further aspect, the BMP may be a mixture of rhBMP-2 and rhBMP-7.

The composition according to present invention may also comprise one or more antibiotic compounds. In principle, the antibiotic may be any agent displaying a binding affinity towards, or accretion to the particulate material. In another aspect, the one or more antibiotic compounds may display little or no accretion to or binding affinity to HA and/or CaP.

Non-limiting examples may be e.g. the antibiotic may be a tetracycline of any kind, such as e.g. tetracycline, chlortetracycline, oxytetracycline, demeclocycline, lymecycline, meclocycline, methacycline, minocycline, rolitetracycline, tigecycline, omadacycline, sarecyclin or any combinations thereof.

In one particular aspect, the antibiotic may be e.g. tetracycline.

Further examples of antibiotics may be any type of antibiotic with a chelating ability towards calcium. This may be in any configuration of a molecule wherein a hydroxyl or amino or phosphonate group are arranged in a vicinal or isolated configuration within the molecule of the antibiotic.

Yet further examples may be e.g. ansamycins. Non-limiting examples thereof are e.g. rifamycins and in particular rifampicin.

Other examples are geldanamycin, herbimycin A, macbecin, natalamycin, streptovaricin or rifamycins in general or any derivatives thereof.

The antibiotic compound may also be a daptomycin.

Consequently, the composition according to the invention may comprise;
i) a carrier or vehicle, wherein the carrier or vehicle may comprise one or more of HA and/or CaP and/or CaS, including any combinations thereof,
ii) one or more BMPs, and optionally
iii) one or more antibiotic compounds.

According to FDA approved dose of BMP (BMP-2) in the context of bone healing is 1500 µg BMP-2/cm³ of bone graft substitute. The Inventors of present invention has surprisingly that by combining BMPs with the carrier according to the invention, the dose of the BMPs may be greatly reduced. A reduction of BMPs in comparison with 1500 µg BMP-2/cm³ (FDA approved dose) according to the invention may be e.g. at least about 5 times, or such as e.g. at least about 10 times, such as e.g. at least about 50 times, such as e.g. at least about 75 times, such as e.g. at least about 100 times, or such as e.g. at least about 120 times. In one aspect, the reduction of the dose of BMPs may be at least about 5 times. In another aspect, the reduction of the dose of BMPs may be at least about 60 times.

Put in another way, the dose of BMPs according to present invention may be e.g. about 300 µg or less BMP/cm³ (volume measured as the volume of the carrier according to the invention), such as e.g. about 150 µg or less BMP/cm³, such as e.g. about 30 µg or less BMP/cm³, such as e.g. about 20 µg or less BMP/cm³, such as e.g. about 15 µg or less BMP/cm³, such as e.g. about 12,5 µg or less BMP/cm³.

Thus, in one aspect, present invention relates to a composition which may comprise
i) a carrier or vehicle, wherein the carrier or vehicle may comprise one or more of HA and/or CaP and/or CaS, including any combinations thereof,
ii) one or more BMPs in an amount of about 300 µg or less BMP/cm³ (volume measured as the volume of the carrier), such as e.g. about 150 µg or less BMP/cm³, such as e.g. about 30 µg or less BMP/cm³, such as e.g. about 20 µg or less BMP/cm³, such as e.g. about 15 µg or less BMP/cm³, such as e.g. about 12,5 µg or less BMP/cm³, and optionally
iii) one or more antibiotic compounds.

In one aspect, the carrier may comprise HA and CaS. As indicated herein, CaS may be in form of the hemi-hydrate or dehydrate form or a mixture thereof.

In another aspect, the carrier may comprise CaP and CaS. As indicated herein, CaS may be in form of the hemi-hydrate or dehydrate form or a mixture thereof.

According to the invention, the ratio between e.g. HA or CaP and e.g. CaS may be in range of about about 1% to about 99.9% between first material (HA and/or CaP):second material (CaS), such as e.g. about 10% to about 90%, about 15% to about 85%, about 20% to about 80%, about 25% to about 75%, about 30% to about 70% , about 35% to about 65%, about 40% to about 60%, about 45% to about 55%, or about 50% to about 50%, about 55% to about 45% , about 60% to about 40%, about 65% to about 35%, about 70% to about 30%, about 75% to about 25%, about 80% to about 20%, about 85% to about 15%, about 90% to about 10%, about 95% to about 5%, or about 99,9% to about 1%. In one aspect, the composition comprises only a first material. In one aspect, the ratio or relationship is about 40% to about 60% between HA or CaP and CaS. In one non-limiting example, the HA (hydroxyapatite) may be in an amount of 40% (wt%) and the CaS (calcium sulphate) may be in an amount of 60% (wt%).

The carrier according to the invention may be pre-formed into an entire implant. In another aspect, the carrier may comprise any particulate from such as e.g. beads, cylindrical rods, pellets or granules of any shape. Moreover, the carrier may be in a form of a paste or any type of semi-fluid form.

The size of the particulate form may vary according to the intended application. In one aspect, the particulate form may be in range of from about 0,1 mm to about 5 mm, such as e.g. about 0,5 mm to about 1 mm, e.g. about 1 mm in size, or about 1.5 mm.

In another aspect, and as mentioned herein, the composition according to the invention may comprise an antibiotic compound or a compound capable of acting as an antibiotic. However, the composition according to the invention may comprise an anti-cancer drug or a drug capable of acting as an anti-cancer drug. Non-limiting examples of this may be e.g. a bisphosphonate such as e.g. zoledronic acid, etidronic acid, clodronic acid, tiludronic acid, pamidronic acid, neridronic acid, olpadronic acid, alendronic acid, ibandronic acid, risendronic acid or any suitable salts thereof. The bisphosphonate be also be a salt wherein a radioactive compound is present or a radioactive compound as ⁹⁹Tc or ²²³Ra or strontium or samarium.

Furthermore, other examples of anti-cancer compounds may be e.g. doxorubicin or other anthracyclines extracted from *Streptomyces* bacterium such as e.g. daunorubicin, epirubicin or idarubicin.

Other agents may be employed are e.g. compounds that are X-ray visible or detectably by standard means such as e.g. fluorescence or MR or compounds used in PET-CT. One example is e.g. compounds comprising ¹⁸F such as e.g. Na¹⁸F or fluorodeoxyglucose (¹⁸F). Other examples of X-ray contrast agent may be e.g. water soluble non-ionic X-ray contrast agents (e.g. iohexol, also known as 5-[*N*-(2,3-Dihydroxypropyl)acetamido]-2,4,6-triiodo-*N,N*'-bis(2,3-dihydroxypropyl)isophthalamide) and/or biodegradable X- ray contrast agents. Thus, the composition according to the invention may comprise on or more compounds detectable by X-ray or MR techniques.

Thus, in one aspect, present invention relates to a composition, which may comprise;
i) a carrier or vehicle, wherein the carrier or vehicle may comprise HA and/or CaP optionally with CaS,
ii) one or more BMPs in an amount of about 300 µg or less BMP/cm³ (volume measured as the volume of the carrier), such as e.g. about 150 µg or less BMP/cm³, such as e.g. about 30 µg or less BMP-2/cm³, such as e.g. about 20 µg or less BMP/cm³, such as e.g. about 15 µg or less BMP-2/cm³, such as e.g. about 12,5 µg or less BMP/cm³, and optionally
iii) one or more anti-cancer drugs.

A further important aspect of the invention is a method of preparing a composition according to the invention.

As mentioned herein, preparing a composition for medical use requires the finished composition to be sterile. The common technique of sterilization entails either heating, irradiation or using etylenoxide. However, when a composition comprise a protein or other sensitive compounds, these methods may prove to be unsuitable as it may destroy or denature e.g. a protein or chemically change a drug substance.

Moreover, when employing carriers that may cure or are intended to cure inside the subject after administration, curing times have to be acceptable (i.e. not too long or short). The inventors of present invention have found that mixing e.g. a BMP with HA and CaS prolongs the curing time to unacceptable levels.

In one aspect, present invention relates to a process of preparing a composition according to the invention. The method may comprise the steps of:
a) providing a carrier comprising one or more of HA, CaP and/or CaS,
b) contacting the carrier in a) with a solution of one or more BMPs during a certain time period,
c) collecting and separating the carrier loaded with one or more BMPs as a result of step b),

In one aspect, the carrier is sterilized by any suitable technique prior to contacting the carrier with a solution of one or more BMPs.

The BMP should preferably be prepared and preserved under sterile conditions.

The process according to the invention may comprise further steps following step c) and such steps may comprise contacting the carrier with a solution of e.g. an anti-cancer drug or an antibiotic compound.

In one aspect, the method may comprise mixing HA and/or CaP and/or CaS and addition of sterile water. This may form a paste that is allowed to stand for a period of time of about 30 seconds, such as e.g. about 1 minute, such as e.g. about 2 minutes, such as e.g. about 3 minutes, such as e.g. about 4 minutes, such as e.g. about 5 minutes. Thereafter, without further mixing, a solution comprising one or more BMPs and/or an antibiotic compound and/or and anti-cancer drug may be added and soaked into the paste. The resulting paste may be e.g. administered to the subject and to the relevant site of treatment in the subject and allowed to cure. Alternatively, the resulting composition may be allowed to cure prior to being administered to the subject.

In one aspect, step b) may comprise contacting the carrier with a solution comprising mixture of e.g. BMPs and one or more antibiotic compounds and/or one or more anti-cancer drugs.

In a particular aspect, step b) may be performed under reduced pressure. Such reduced pressure may be below normal atmospheric pressure, i.e. below 1 atm (about 1 bar) measured at NTP. In one aspect, the reduced pressure may be about 0.5 bar or less, such as e.g. about 0.2 bar or less. The inventors of present invention have surprisingly found that by contacting the carrier according to the invention at reduced pressure a very high loading of BMPs, antibiotics and anti-cancer drugs into the carrier is observed.

The contacting time or time period referred to in step b) may be in order of 1 minute or more, such as e.g. about 5 minutes, e.g. about 10 minutes, e.g. about 15 minutes, e.g. about 20 minutes, e.g. about 25 minutes, e.g. about 30 minutes, e.g. about 60 minutes etc.

In one aspect, after the time period as mentioned above for step b), the obtained carrier is separated from the solution. Optionally, the carrier is rinsed with water or saline. The rinsing procedure may be repeated several times. Alternatively, the carrier may be allowed to dry after being collected and separated from the solution.

As mentioned herein, present invention also relates to use of the composition according to the invention in bone healing or bone regeneration.

Specifically, the use according to the invention relates to administering the composition according to the invention into the site of a subject wherein bone healing or bone regeneration is needed. Non-limiting examples may be use or application in the context of spinal fusion, healing of any type of fracture in e.g. femur or hip etc.

Moreover, present invention also relates to the composition according to the invention for use in bone healing or bone regeneration. Thus, to composition for use may entail administering the composition according to the invention into the site of a subject wherein bone healing or bone regeneration is needed. Non-limiting examples may be use or application in the context of spinal fusion, healing of any type of fracture in e.g. femur or hip etc.

In another aspect, present invention relates to a method of treatment. The treatment may comprise administering the composition according to the invention to a subject in need thereof. Specifically, the method may comprise administering the composition according to the invention into the relevant site(s) of the subject for e.g. bone healing or bone regeneration and may comprise, but not excluding other areas of application, spinal fusion healing of any type of fracture in e.g. femur or hip etc.

### Experimental section

The following experiments are intended to illustrate the invention. However, it is to be understood that the experiments are in no way to be regarded as limiting the scope of the invention. "Cerafill" may be regarded as the carrier or composition according to the invention.

### Example 1:

Methods: 500 mg of 39.99 wt% hydroxyapatite (HA) and 59.98 wt% calcium sulfate hemihydrate (CaS) and 0.024 wt% calcium sulfate dihydrate (CSD) was weighed in two separate wells of a 24-well plate. In the first well, 0.19 mL of 0.9 wt% NaCl was added, followed by mixing with a spatula for 30 s. At this stage the plate was gently tapped to achieve a flat surface of the paste. In the second well, 40 microgram of BMP-2 was first dissolved in 0.19 mL of 0.9 wt% saline. This solution was then added to the second well containing CaS/HA/CSD powder, mixed for 30 sec and the plate gently tapped. Following this, the final setting time (starting from the initiation of mixing) of the material in both wells was studied using a Gilmore needle apparatus.

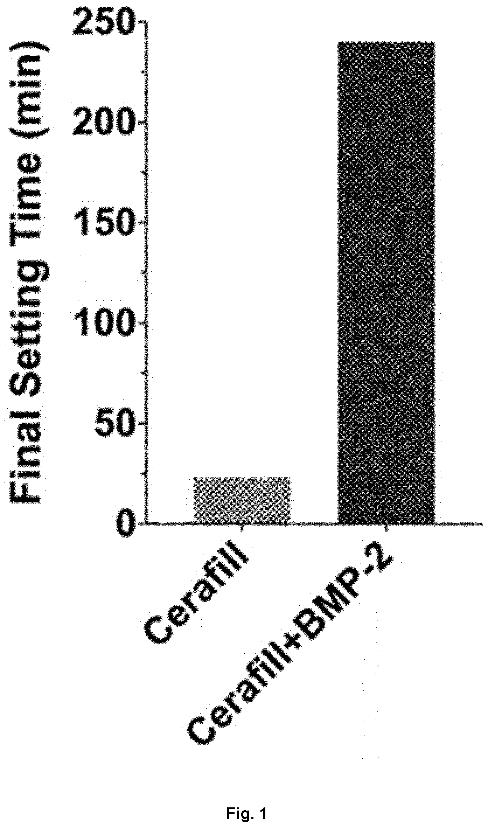

Results: The biomaterial without the BMP-2 had a final setting time of 23 min (illustrated in Figure 1). Biomaterial mixed with BMP-2 exhibited a significant delay in setting time and did not set within the tested 240 min. It is to be noted that this is observed despite the fact that a curing accelerator is added (calcium sulfate dihydrate).

Conclusion: Addition of BMP-2 to a biphasic material in the paste phase causes a significant delay in the setting time of material. This significant increase (>10 times) in the setting time indicates that it is not favorable to include the protein in the paste form in an operating room setting.

### Example 2:

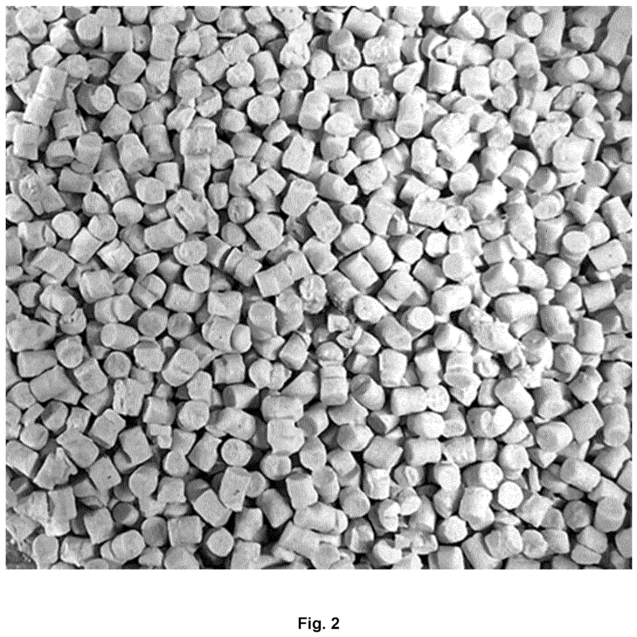

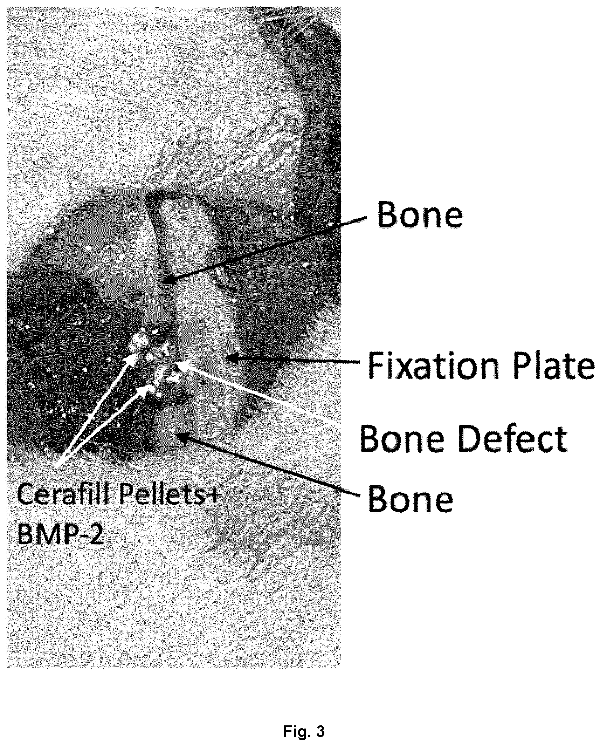

Methods: Beads comprising 40 wt% hydroxyapatite and 60 wt% calcium sulfate hemihydrate were casted by mixing 1 g of HA/CaS powder mix with 0.38 mL of 0.9 wt% NaCl (saline) in a silicone bead mold with 1 mm diameter and 1 mm height (illustrated in Figure 2). After 15 min, the beads were removed and sent for gamma sterilization before animal experimentation. Sprague-Dawley male rats aged 10-12 weeks old were used for the experiment and a critical femur defect model was used. A 5-6 mm piece of the mid-diaphyseal femoral bone was removed and stabilized with a PEEK plate and 4 stainless steel screws. The defect was then either left empty or filled with Cerafill beads (illustrated in Figure 3).

A total of 40 pre-set pellets having a total volume of 0.0316 cm³ were soak loaded with 10 microgram BMP-2, 5 min prior to implantation. In comparison with the FDA approved Infuse Bone Graft which is used at a dose of 1500 microgram BMP-2/cm³ bone graft substitute, the current dose used in the rat study indicates a 5-time dose reduction when considering the volume of the bone graft material used. After a healing period of 6-weeks, the animals were scanned using a micro-CT scanner in order to evaluate the level of radiological healing of the femoral defect.

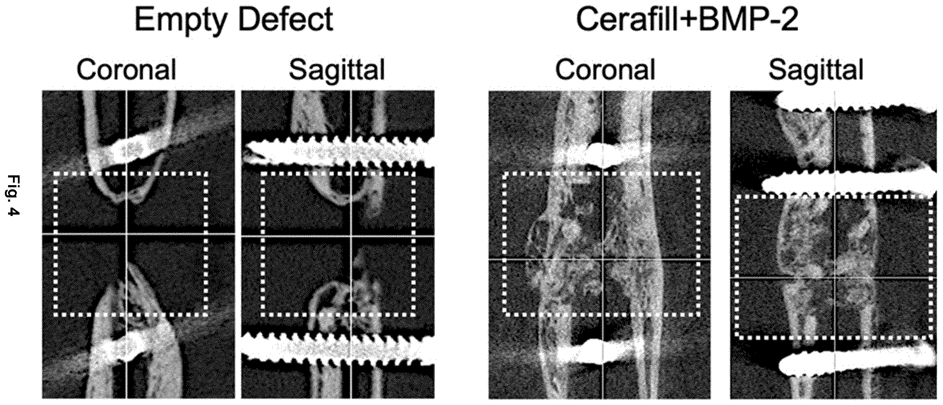

Results: Empty defect did not heal after 6-weeks as seen from the micro-CT images (illustrated in Figure 4). The defect in the Cerafill+BMP-2 group was completely bridged both in the coronal and the sagittal planes at the 6-weeks' time point.

Conclusion: The ceramic material (Cerafill Pellets) soak loaded with low dose BMP-2 surprisingly performs at par with if the protein was added into the material from the start (during the paste phase) as shown by us in an earlier study. This is an important finding and makes the application of BMP-2 feasible in an operating room setting. As it can be seen in example 1, the setting time is delayed significantly when the protein is added to the ceramic material during the paste phase. Furthermore, the ceramic paste is more likely to get diluted with the blood at the time of application leading to uncontrolled wash out and release of BMP-2.

### Example 3:

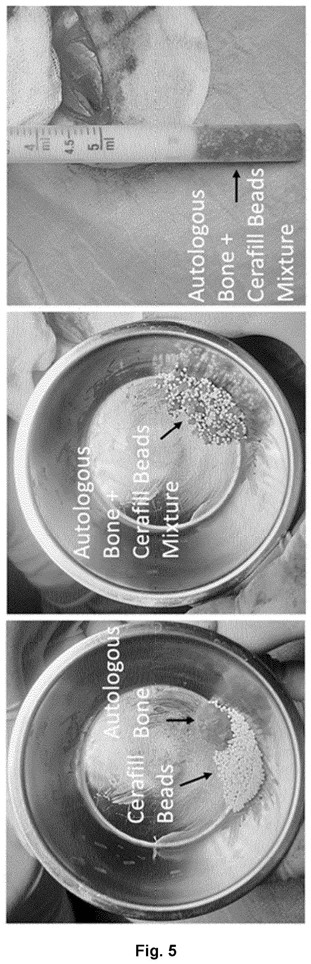

Methods: A well-established rabbit posterolateral spinal fusion model was used in this experiment. Skeletally mature female New Zealand white rabbits aged between 8-10 months were operated on under general anesthesia. Lumbar spine was exposed in a layered fashion until the transverse processes of lumbar vertebra L4 and L5 were bilaterally exposed under radiographic guidance. The transverse processes of both vertebral bodies were decorticated using a pneumatic drill connected to a 1 mm drilling burr. Once all 4 transverse processes were decorticated and punctate bleeding could be observed, the animals were treated as follows: 1) 2 cm³ of morselized (< 5 mm) autogenous bone harvested from the iliac crest placed on each side of the vertebral body (i.e. total 4 cm³ of grafting material) as a positive control, 2) 2 cm³ of 50:50 volume % mixture of autologous bone (1 cm³) and pre-set Cerafill pellets of 1 mm diameter and 1 mm height (1 cm³) placed on each side (i.e. total of 2 cm³ autologous bone and 2 cm³ Cerafill pellets) and 3) 2 cm³ Cerafill pellets soak loaded with 50 microgram rhBMP-2 placed on either side of the vertebral body (i.e. total of 4 cm³ Cerafill beads containing 100 microgram BMP-2). The grafting materials in groups 1-3 were molded into a cylindrical shape using a 1 cm diameter open bore syringe and a total of two cylinders were made in order to be applied on either side of the spine. In group 2, the Cerafill pellets were mixed with autogenous bone graft thoroughly in a sterile bowl after which the mixture was filled into an open bore syringe before application (illustrated in Figure 5).

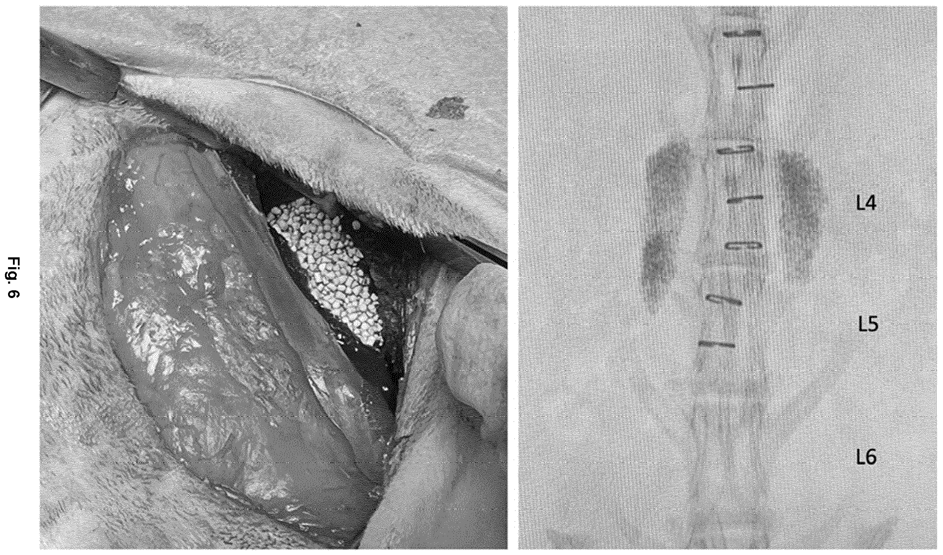

In group 3, 2 cm³ Cerafill pellets were first added to a petri-dish followed by soak loading the beads with 600 microliter saline containing 50 microgram BMP-2. All liquid was absorbed by the beads without any remaining excess fluid. The beads were then loaded into an open bore syringe as described above and the process was repeated for the other side of the spine. BMP-2 loaded beads were applied to the animals within 15 min after being soak loaded with the BMP-2. In order to make the Cerafill pellets stick to each other better, 100 microliter of peripheral blood was applied to the open bore syringe prior to introducing the grafting material into the animal. In all groups the grafting material shaped as a cylinder was applied as a bridge between the bleeding L4 and L5 transverse processes (illustrated in Figure 6).

In order to evaluate healing responses, the animals were subjected to in-vivo radiography using a C-arm to confirm bilateral bridging of the transverse processes.

### Results:

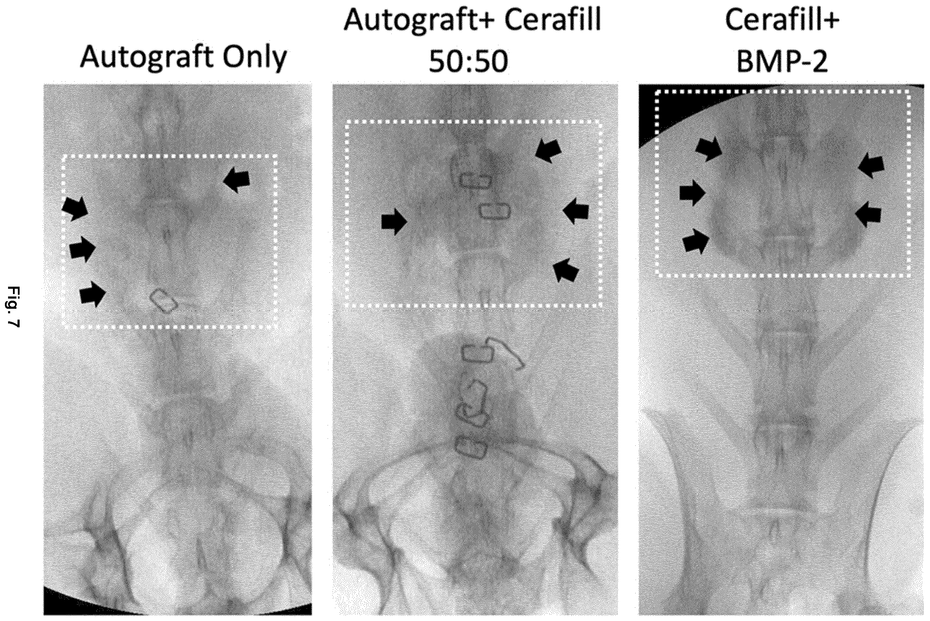

In the groups containing autograft (Group 1) or 50:50 vol% autograft:Cerafill unilateral less dense bone fusion could be observed at the 6-week time point whereas the Cerafill+BMP-2 displayed a robust bilateral bony fusion spanning the entire length of the transverse processes (illustrated in Figure 7).

Conclusion: In a rabbit posterolateral spinal fusion model, we show that 50 microgram BMP-2 on each side in the CaS/HA beads (total 100 microgram on both sides) leads to spinal fusion already at 6-weeks. Total of 4 cm³ of grafting material (Cerafill beads) were soak loaded with 100 microgram BMP-2 corresponding to a dose of 25 microgram BMP-2/cm³ grafting material. FDA approved BMP-2 dose equates to 1500 microgram BMP-2/cm³ indicating that the dose used in the rabbit study is 60 times lower than the currently used BMP-2 dose. The BMP-2 group was compared with either a 100% autograft group or to autograft: Cerafill mixture at a 50:50 volume ratio. Radiographic results show that the BMP-2 group was superior to only autograft and autograft combined with a 50 % extender.

### Example 4:

Methods: Cerafill pellets of 4.8 mm were prepared and incubated with 1 mL of antibiotics tetracycline (TET) and rifampicin (RIF) both having a concentration of 4 microgram/mL, which equates to their serum concentration achieved in humans. Cerafill pellets were incubated in the antibiotic containing solutions for either 1 min or 15 min or 1 h after which each pellet was washed 3 times with saline in order to mimic the in-vivo situation where the implant comes in contact with blood. After washing, the pellets were dried on a cotton gauze for 1 min after which they were placed on a Muller-Hinton agar plate containing a culture of *S*. *aureus.* The samples were incubated at 37 °C for 24 h following which zone of inhibition diameters were measured using digital photos.

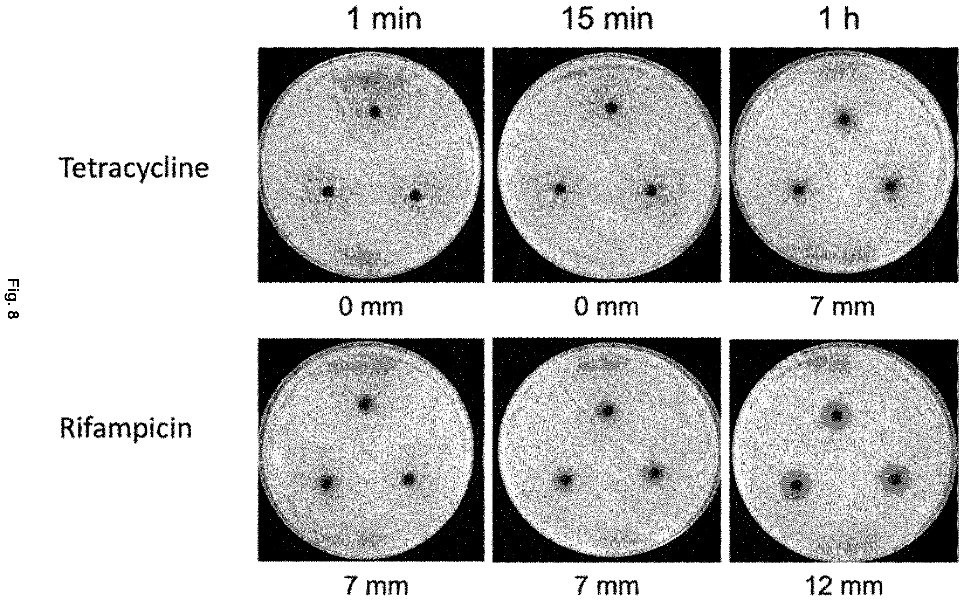

Results: In the case of TET, after incubating the material in a very low concentration of antibiotic, a zone of inhibition (ZOI) was not be observed at 1 and 15 min, whereas a 7 mm ZOI could be observed at 1 h. In the case of RIF, the Cerafill pellets were protected from bacterial colonization already at 1 and 15 at which time the ZOI size reached 7 mm, which further increased to 11 mm at 1 h (illustrated in Figure 8).

Conclusion: A microporous biphasic ceramic material can be protected by bacterial colonization by soak loading of antibiotics.

### Example 5:

Methods: Cerafill pellets of 2.3 mm were prepared and placed in a tube containing 1 ml of 2 mg/mL doxorubicin (DOX) solution. A total of 3 pellets were placed in each tube for 2 min, after which the pellets were removed and the concentration of DOX in the supernatant was measured.

Result: The measured concentration of DOX in the solution before the addition of CaS/HA pellet was 2 mg/mL. 2 min after incubation with Cerafill pellet, the concentration of DOX in the solution was 1.7 mg/mL indicating that 15% of the drug could be loaded onto the ceramic material already after 2 min.

Conclusion: Soak loading can be used for loading a cytotoxic drug on a biphasic ceramic material.

### Example 6:

Methods: 2.3 mm beads of Cerafill were molded in a silicone mold and allowed to set for 30 min. Immediately after setting, the beads were dipped in separately placed in 2 mL Eppendorf tubes followed by pipetting 0.5 mL of saline solution in each tube. Each tube contained only one pellet. One set of tubes were placed in a vacuum chamber run by an ejector driven vacuum pump at an under pressure of 0.2 bar while the other set of tubes were placed at atmospheric pressure. Pellets were removed from their respective tubes at 1, 2, 4 and 8 min and the volume of remaining saline in each tube was measured using a pipette. As a continuation of this experiment, another two 2.3 mm pellets were placed separately in 2 Eppendorf tubes containing 0.5 mL Safranin O staining solution. One of the tubes was inserted in a vacuum chamber with an under pressure of 0.2 bar for 8 min. The other tube was placed at room temperature. At the end of 8 minutes, the pellets were harvested and cut into two parts to macroscopically observe the stain penetration in the microporous Cerafill beads.

### Results:

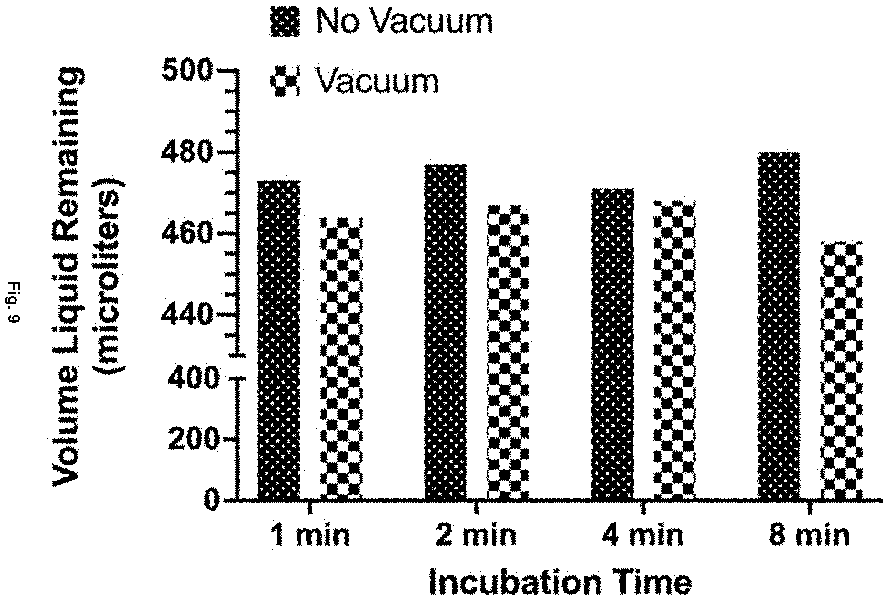

The pellets in the group without vacuum absorbed maximum liquid within the 1st min of being incubated in the saline solution after which increase in incubation time did not correspond to an increase in the volume of liquid absorbed by the material. In the group with vacuum, the pellets generally soaked more liquid but this change was more noticeable at 8 min where in the volume of the liquid absorbed by the pellet under vacuum was twice the volume without vacuum (illustrated in Figure 9).

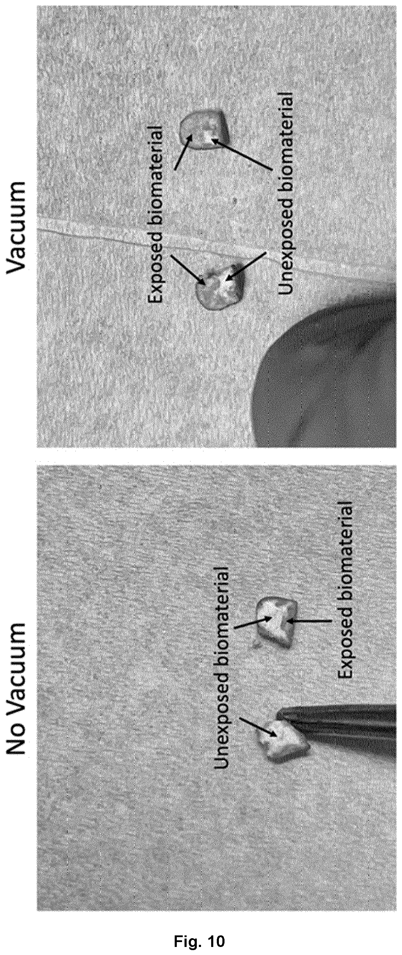

These results were further validated by the experiment conducted using histological stain Safranin O that clearly showed deeper penetration into the material with vacuum when compared with without vacuum (illustrated in Figure 10).

### Conclusion:

Application of under pressure enhances penetration of fluids in a microporous biphasic ceramic material to a degree higher than expected.

## Claims

1. A composition comprising:
a) a carrier comprising hydroxyapatite (HA) and/or calcium phosphate (CaP) and/or calcium sulphate (CaS),
b) one or more bone morphogenic proteins (BMPs) in an amount of about 300 µg or less BMP/cm³ (volume measured as the volume of the carrier in a)), such as e.g. about 150 µg or less BMP/cm³, such as e.g. about 30 µg or less BMP/cm³, such as e.g. about 20 µg or less BMP/cm³, such as e.g. about 15 µg or less BMP/cm³, such as e.g. about 12,5 µg or less BMP/cm³.

2. The composition according to the invention, wherein one material in the carrier is calcium sulphate in any state of hydration or crystal configuration, or wherein the carrier is a mixture of HA and CaS, or a mixture of CaP and CaS, or a mixture of HA, CaP and CaS.

3. The composition according to the preceding claims, wherein the BMP is rhBMP-2 and/or rhBMP-7.

4. The composition according to the preceding claims, wherein the composition further comprises an antibiotic compound and/or an anti-cancer drug.

5. The composition according to the preceding claims, wherein the antibiotic compound is one or more of tetracycline, chlortetracycline, oxytetracycline, demeclocycline, lymecycline, meclocycline, methacycline, minocycline, rolitetracycline, tigecycline, omadacycline, sarecyclin or any combinations thereof, or an ansamycins such as e.g. rifamycins and in particular rifampicin, or, geldanamycin, herbimycin A, macbecin, natalamycin, streptovaricin or rifamycins in general or any derivatives thereof.

6. The composition according to the preceding claims, wherein the drug is one or more of a bisphosphonate such as e.g. zoledronic acid, etidronic acid, clodronic acid, tiludronic acid, pamidronic acid, neridronic acid, olpadronic acid, alendronic acid, ibandronic acid, risendronic acid or any suitable salts thereof, or the anticancer drug is doxorubicin, daunorubicin, epirubicin or idarubicin.

7. The composition according to the preceding claims, wherein ratio between e.g. HA or CaP and e.g. CaS may be in range of about about 1% to about 99.9% between first material (HA and/or CaP):second material (CaS), such as e.g. about 10% to about 90%, about 15% to about 85%, about 20% to about 80%, about 25% to about 75%, about 30% to about 70% , about 35% to about 65%, about 40% to about 60%, about 45% to about 55%, or about 50% to about 50%, about 55% to about 45% , about 60% to about 40%, about 65% to about 35%, about 70% to about 30%, about 75% to about 25%, about 80% to about 20%, about 85% to about 15%, about 90% to about 10%, about 95% to about 5%, or about 99,9% to about 1%.

8. The composition according to the preceding claims, wherein the composition is in form of a paste, pellets, granules, beads or rods.

9. The composition according to the preceding claims, wherein the particle sizes of HA and/or CaP have particles in size of about 0.5 µm to about 50 µm and 40 nm to about 100 nm or a mixture of said particle sizes.

10. The composition according to the preceding claims, wherein the further comprises agents that are X-ray visible or detectably by standard means such as e.g. fluorescence or MR or compounds used in PET-CT, wherein the agents are compounds comprising ¹⁸F such as e.g. Na¹⁸F or fluorodeoxyglucose (¹⁸F), or iohexol, also known as 5-[N-(2,3-Dihydroxypropyl)acetamido]-2,4,6-triiodo-*N,N*'-bis(2,3-dihydroxypropyl)isophthalamide, and/or biodegradable X- ray contrast agents.

11. A method of preparing the composition according to the preceding claims, the method comprising;
a) providing a carrier comprising one or more of HA and/or CaP, and/or CaS,
b) contacting the carrier in a) with a solution of one or more BMPs and/or one or more antibiotic compounds and/or one or more drugs according to claims 4-6, during a certain time period, wherein contacting takes place under reduced pressure,
c) separating the obtained carrier in step b) from the solution,

12. The method according to claim 11, where the reduced pressure is about 0.5 bar or less, such as e.g. about 0.2 bar or less.

13. The method according to claims 11-12, wherein time period referred to in step b) may be in order of 1 minute or more, such as e.g. about 5 minutes, e.g. about 10 minutes, e.g. about 15 minutes, e.g. about 20 minutes, e.g. about 25 minutes, e.g. about 30 minutes, e.g. about 60 minutes etc.

14. The compound according to claims 1-10, for use in bone healing or bone regeneration in a subject in need thereof.

15. A method of treating a subject in need of bone healing or bone regeneration, the method comprising administering the composition according to any of claims 1-10 into the subject and into the relevant site for bone healing or bone regeneration.
